# EUROPEAN PATENT APPLICATION

(11) **EP 1 662 417 A1**
(43) Date of publication of application: **31.05.2006**
(21) Application number: 05257375.5
(22) Date of filing: 28.11.2005
(51) Int. Cl.: G06F 19/00, G06F 17/30

(54) **System and method for recording medical image data on digital recording media**

(30) Priority: 27.11.2004 US 631263 P
(71) Applicant: McDonough Medical Products Corporation, Deerfield IL 60015 (US)
(72) Inventor: Langhofer, Laurn R., Auburn California 95603 (US); Preston, Andrew, Orangevale California 95662 (US)
(74) Representative: Lawrence, John

(57) **Abstract**

A system and method for recording medical image data onto a digital recording media. The system and method comprising a medical imaging modality interface (28) for receiving a plurality of video images from a medical imaging modality. A video capturing device (42) is coupled to the medical imaging modality for capturing the plurality of video images from the medical imaging modality and converting the plurality of video images into a plurality of digital images. A media writer (44) is coupled to the video capturing device (42) for recording the plurality of digital images onto a removable media. A computer (46) having a CPU (48) installed thereon is coupled to the medical imaging modality interface (28), the video capturing device (42) and the media writer (44) for controlling operation of the system. And a user interface (26) is coupled to the computer (46), the medical imaging modality interface (28), the video capturing device (42) and the media writer (44) for allowing user input in controlling operation of the system and method.

## Description

### CROSS-REFERENCE TO RELATED APPLICATIONS

This application is based on and claims the benefit of U.S. Provisional Application No. 60/631,263, filed November 27, 2004, and incorporated herein by reference.

### BACKGROUND OF THE INVENTION

The present invention relates generally to a system and method for recording medical video image data on portable digital recording media such as CDs or DVDs. More particularly, the present invention relates to a system and method for receiving medical image and video data, processing the medical image and video data, and transmitting the medical image and video data to be recorded on a portable digital recording medium.

X-ray film has been the predominant multipurpose medium for the acquisition, storage and distribution of medical images. However, the storage and distribution of film often requires considerable expense and storage space. X-ray imaging devices are followed by a variety of different methods of imaging body tissue, such as ultrasound, nuclear medicine, computed tomography (CT) and magnetic resonance imaging (MRI). X-ray devices are giving way to digital systems in which computer screens replace film. Digital picture archiving and communication systems (PACS) are replacing film libraries and helping radiologists work more efficiently.

Today's modem health care facilities utilize computer-aided imaging devices such as ultrasound, computed tomography (CT) and magnetic resonance imaging (MRI). These devices can generate hundreds of images in a matter of seconds. Many health care facilities still require the images generated to be printed on film for storage and distribution. To print complete sets of medical images from these imaging devices requires considerable expense in film material, storage space and management

Some radiology departments of modern health care facilities have installed digital image storage and management systems known as PACS (Picture Archive Communication Systems). PACS are capable of storing a large amount of medical image data in digital form. To ease the communication of digital data, the DICOM (Digital Imaging and Communications in Medicine) standard was developed by ACR-NEMA (American College of Radiology-National Electrical Manufacturer's Association) for communication between medical imaging devices and PACS. PACS store medical image data in DICOM format. In addition to the examined images, patient demographics, and exam information such as patient name, patient age, exam number, exam modality, exam machine name, and exam date can also be stored and retrieved in a DICOM compatible data format. A DICOM file typically stores patient and exam information in the header of the file, followed by exam images.

Other prior art methods of storing medical images and video is through the use of S-VHS video tape for playback on VCRs and magneto optical (MO) disk media. However, all of these prior art systems and methods of archiving video images and videos require specialized playback or viewing hardware and have other problems associated with them as well.

### SUMMARY OF THE INVENTION

The present invention is a system and method for recording medical image data onto a digital recording media. The system and method comprising a medical digital recorder (MDR) having a medical imaging modality interface for receiving a plurality of video images from a medical imaging modality. A video capturing device is coupled to the medical imaging modality for capturing the plurality of video images from the medical imaging modality and converting the plurality of video images into a plurality of digital images. A media writer is coupled to the video capturing device for recording the plurality of digital images onto a renlovable media. A computer having a CPU installed thereon is coupled to the medical imaging modality interface, the video capturing device and the media writer for controlling operation of the system. A user interface is coupled to the computer, the medical imaging modality interface, the video capturing device and the media writer for allowing user input in controlling operation of the system and method.

The present invention provides for digital medical image or video data to be recorded on a potable digital recording medium such as a CD or DVD. The CD or DVD containing the medical image or video data that can be distributed to physicians, hospitals, patients, insurance companies, etc. The system and method also allows for medical image or video data to be placed on a CD or DVD along with a viewing program, so that a user can use any computer having a CD or DVD reader to view the medical image or video data that has been recorded on the CD or DVD.

The present invention is specifically designed to record medical video image data from the highest resolution output available from diagnostic imaging systems. Analog format video data from the imaging systems is digitized by the MDR and written to a recordable media such as CD or DVD media in a medical digital compliant format such as DICOM (Digital Imaging and Communications in Medicine). Alternatively, the video image data can be written to a disk in MPEG format for playback on any DVD player. The present invention incorporates a plurality of medical imaging specific features, which differentiate it from consumer and/or industrial video to DVD recorders.

The MDR is an analog capture device and a digital video recorder that captures the full spectrum of medical image video using a high grade format and writes them to a CD or DVD. The MDR is capable of recording images from any imaging modality that has a video output. The unique features of the invention include the MDR's user interface and its video capture capabilities.

**[0010]** In one aspect of the invention, the MDR is designed to provide portable CD and DVD recording technology for DICOM image management. The MDR receives and writes original DICOM images to either CD or DVD media from any imaging modality or from a Picture Archiving and Communication System (PACS) network The present invention is a medical grade CD and DVD recorder specifically designed for use in both static and mobile imaging applications.

The MDR preferably records DICOM images for review, treatment planning, referrals, patient copies, and archiving. The MDR receives and writes true DICOM images from any medical imaging modality or PACS network to DVD or CD media. The MDR records DICOM images in their original digital format, so there is no resolution loss, no matter how many copies are made. The MDR system includes a DICOM viewer application program that is automatically installed on each disk, allowing for easy image viewing on any personal computer.

The MDR provides a compact integrated system for use with both portable imaging systems and fixed imaging modalities. It provides a great cost savings of recording images to CDs or DVDs compared to other prior art alternatives. It also provides versatility for image distribution.

With its compact footprint, the MDR fits easily into portable ultrasound and C-arm systems. The MDR also allows users to copy images during or after a procedure and use them immediately for referrals, patient records or viewing in other areas of a health care facility. It is also useful on fixed modalities like CT, MRI or cardiology systems where a secondary image capture solution is desired. It has a low profile design that takes up minimal space, and provides easy connectivity to any imaging modality and PACS network.

The MDR system is designed to work efficiently with minimal operator interaction. It is always capture-ready and provides both multiple archive and single patient modes of operation. In the archive mode of operation, the MDR records images continuously until the disk is either full or removed by the operator. Unrecorded images are stored on the MDR's hard drive until new removable media is inserted. A DICOM Part 10 compatible directory allows easy search and retrieval of individual records contained on each disk. MDR could also be used as a redundant archive system to facilitate HIPAA compliance, or even as a complete digital image archive for small departments or imaging centers. The single patient mode of operation records one patient record per disk along with an optional DICOM viewer application program. The single patient mode of operation is ideal for creating files for referring physicians or patient communications, and allows staff to view individual exams on any available personal computer, recurring reliance on DICOM viewing stations.

As mentioned above, the MDR system includes an optional DICOM viewer application program that can be burned into each disk for playback. It is completely intuitive so the user can retrieve images quickly for immediate visualization and manipulation of the complete DICOM data. Some unique features included with the DICOM viewer application program include file export, e-mail and printing utilities.

Cost reduction and increased storage capacity are major benefits of the MDR system. An imaging exam recorded on a CD or DVD is about one-fourth the cost of duplicate flat film containing the same data. A DVD can record up to 5000 images or about two and one-half minutes of uncompressed streaming video at a fraction of the cost compared to S-VHS (Super VHS) tape or MO (magneto optical) disk media, and does not require the addition of specialized playback or viewing hardware.

Also, DVDs and CDs can be produced much more quickly and economically than other prior art archiving options. CD and DVD media can be produced at one-forth the cost of film, one-tenth the cost of DAT (Digital Audio Tape) tape and one-fifth the cost of S-VHS tape. Plus, disks can record an amazing amount of data. More than 700 single images on one CD, and more than 5,000 single images or equivalent streaming video on one DVD.

Various other features, objects, and advantages of the invention will be made apparent to those skilled in the art from the following detailed description, claims, and accompanying drawings.

### BRIEF DESCRIPTION OF THE DRAWINGS

FIG. 1 is a schematic diagram illustrating the medical digital recorder used in two different applications in accordance with the present invention;

FIG. 2 is a block diagram of the medical digital recorder of the present invention;

FIG. 3 is a diagram illustrating an example front panel of the medical digital recorder of the present invention;

FIG. 4 is a diagram illustrating an example rear panel of the medical digital recorder of the present invention;

FIG. 5 is a diagram of the display of the medical digital recorder of the present invention;

FIGS. 6A-6C are diagrams of the display of the medical digital recorder of the present invention illustrating the recording process during an archive mode;

FIGS. 7A-7C are diagrams of the display of the medical digital recorder of the present invention illustrating the recording process during a single patient mode; and

FIG. 8 is an application data flow diagram of the medical digital recorder of the present invention in a DICOM application.

### DETAILED DESCRIPTION OF THE INVENTION

In medical imaging, Picture Archiving and Communication Systems (PACS) are computers or networks dedicated to the storage, retrieval, distribution and presentation of medical images. Full PACS handle images from various modalities, such as fluoroscopy, ultrasonography, radiography, magnetic resonance imaging (MRI), positron emission tomography (PET), computed tomography (CT) and so on. PACS replaces hard-copy based means of managing medical images, such as film archives. It expands on the possibilities of such conventional systems by providing capabilities of off-site viewing and reporting. Typically a PACS network consists of a central server which stores a database containing the images. This server is connected to one or more clients via a LAN or a WAN, which provide and/or utilize the images. Client workstations can use local peripherals for scanning image films into the system, printing image films from the system and interactive display of digital images. Modem radiology equipment feeds images directly into PACS in digital form. The medical images are stored in an independent format. The most common format for image storage is DICOM (Digital Imaging and Communications in Medicine).

DICOM is a comprehensive set of standards for handling, storing and transmitting information in medical imaging. It includes a file format definition and a network communication protocol. DICOM was developed to enable integration of scanners, servers, work stations and network hardware from multiple vendors into a picture archiving and communication system. DICOM files consist of a header with standardized as well as free-form fields and a body of image data. A single DICOM file can contain one or more images, allowing for the storage of volumes of images and/or video animations. Image data can also be compressed using a variety of standards.

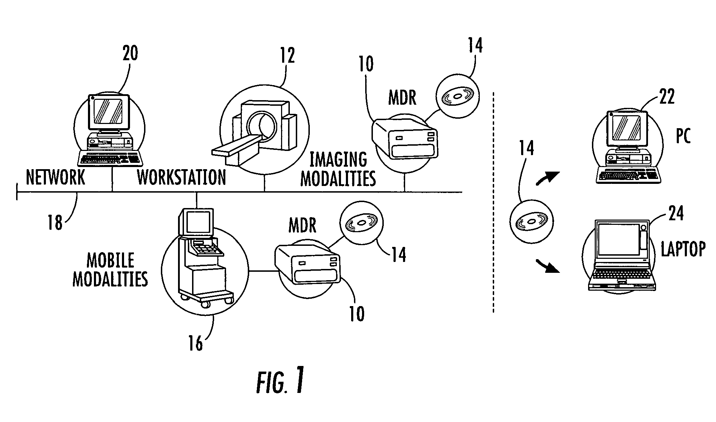

FIG. 1 is a schematic diagram of a medical digital recorder (MDR) 10 used in two different applications in accordance with the present invention. The MDR 10 is designed specifically for medical imaging applications, and not as an offshoot of comrnercial or home entertainment components. The MDR 10 conveniently integrates with existing imaging modalities such as ultrasound, computed tomography (CT) and magnetic resonance imaging (MRI) for recording the medical images and video to recordable media such as CDs or DVDs 14 in a medical digital compliant format such as DICOM (Digital Imaging and Communications in Medicine). Alternatively, the video image data can be written to a disk in MPEG format for playback on any DVD player.

The MDR 10 provides a compact integrated system for use with both portable or mobile imaging systems 16 and fixed imaging modalities 12. For fixed imaging modalities 12, the MDR 10 is preferably coupled to a network 18 to interface with the imaging modality 12 and a workstation 20 that are also coupled to the network 18 for recording medical images and video from the fixed imaging modality 12 onto a CD or DVD 14. For mobile imaging modalities 16, the MDR 10 is preferably coupled to the mobile imaging modality 16 for recording medical images and video from the mobile imaging modality 16 onto a CD or DVD 14. The CD or DVD 14 may then be taken to any personal computer 22 or any laptop computer 24 for playback and viewing.

The MDR of the present invention provides a great cost savings of recording medical images and video to CDs or DVDs compared to other prior art alternatives. It also provides versatility for image distribution. For the highest quality playback, imaging exams can be recorded to DVDs or CDs using the DICOM format and organized for quick retrieval and review from any personal computer or personal computing device using a DICOM viewer or a PACS workstation.

The MDR has a compact footprint, enabling it to fit easily into portable imaging modality systems, such as portable ultrasound or C-arm systems. The MDR also allows users to copy images during or after an imaging procedure and use them immediately for refemals, patient records or viewing in other areas of a health care facility. It is also useful on fixed modalities like CT, MRI or cardiology systems where a secondary image capture solution is desired. Its low profile design takes up minimal space, and its easy connectivity makes it a simple addition anywhere on a PACS network.

The MDR is intended for use with medical imaging equipment and workstations that require storing cine loops to a removable media. Images written to removable media may be viewed on a personal computer with the appropriate CD or DVD player. A DICOM image viewer may also be written to the CD or DVD allowing image review on personal computers that do not have a DICOM viewer application.

The MDR is designed to provide portable CD and DVD recording technology for DICOM image management. The MDR receives and writes original DICOM images to either CD or DVD media from any imaging modality or from a PACS network. It is a medical grade CD and DVD recorder specifically designed for use in both static and mobile imaging applications. The MDR provides high resolution video recording of dynamic and static medical images in a DICOM recording format.

The MDR can be configured with CD only, DVD only, or a combination CD/DVD drive. The compatible media preferably includes CD-R, CD-RW, DVD+R or DVD+RW media. The present invention provides quick, efficient image capture from a DICOM network or directly from the imaging modality. It can be installed as a network device in any clinical environment. It makes any personal computer monitor or laptop personal computer monitor an image review station. The MDR is always in receive mode, meaning it is ready to capture imaging data at all times. It is able to record individual (single patient mode) or multiple (archive mode) imaging exams to a disk.

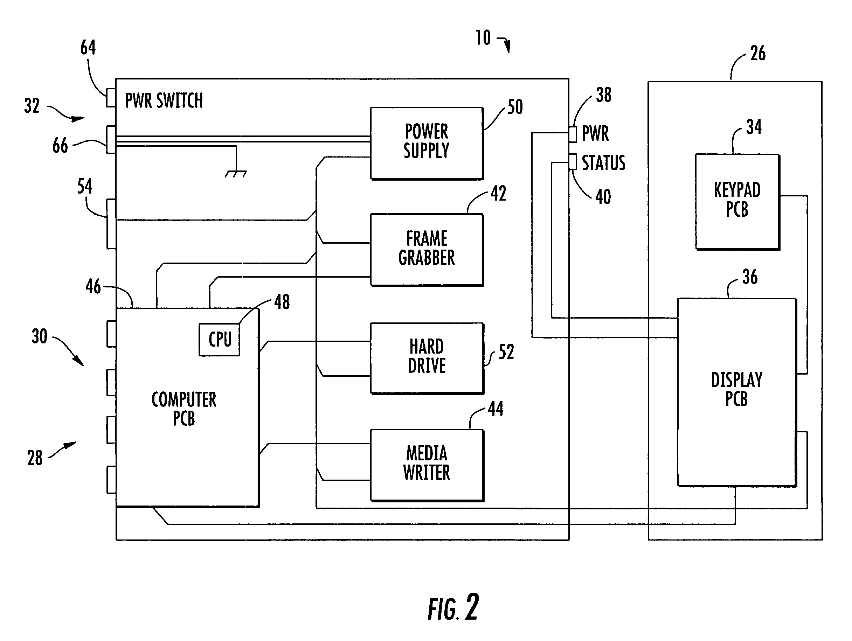

FIG. 2 is a block diagram of the medical digital recorder 10 of the present invention. The MDR 10 includes a user interface 26, and an imaging modality interface 28, a network interface 30, and a power interface 32. The user interface 26 preferably includes a keypad printed circuit board 34 with electronic circuitry thereon and a display printed circuit board 36 with electronic circuitry thereon. Also included in the user interface 26 are a power indicator 38 and a status indicator 40.

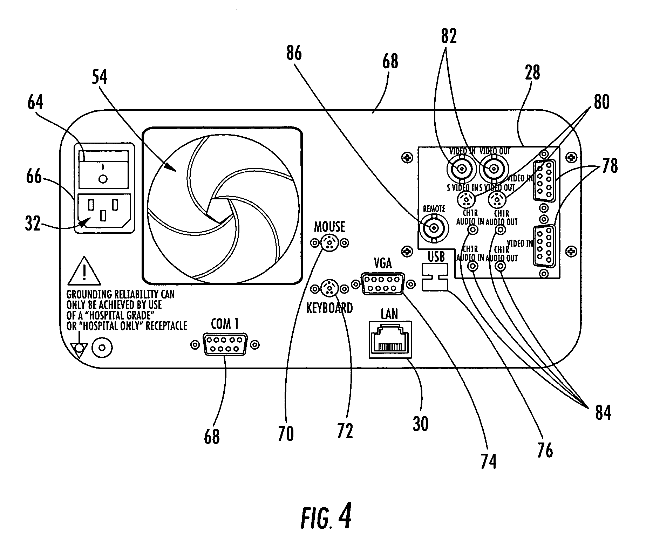

The imaging modality interface 28, network interface 30, and power interface 32 are best seen in FIG. 4, which illustrates the rear panel of the MDR 10. The imaging modality interface 28 receives a plurality of video signals from a medical imaging modality. The MDR 10 includes a frame grabber or a video capturing device 42 that is coupled to the medical imaging modality for capturing a plurality of video images from the medical imaging modality and converting the plurality of video images into a plurality of digital images. The MDR 10 further includes a media writer 44 coupled to the video capturing device 42 for recording the plurality of digital images onto a removable media. The MDR 10 also includes a computer 46 having a CPU 48 installed thereon coupled to the imaging modality interface 28, the video capturing device 42 and the media writer 44 for controlling operation of the system. The user interface 26 is preferably coupled to the computer 46, the imaging modality interface 28, the video capturing device 42 and the media writer 44 for allowing user input in controlling operation of the system. The MDR 10 further includes an internal power supply 50 receiving power from an external AC power source, an internal hard drive 52 for storing the plurality of digital images thereon, and a fan 54 for cooling the internal structure of the MDR 10.

The internal power supply 50 is coupled to and provides power to the computer 46, frame grabber 42, hard drive 52, media writer 44, and display 36. The display 36 is coupled to the keypad 34, power 38 and status 40 indicators, power supply 50 and computer 46. The media writer 44 is coupled to the power supply 50 and the computer 46. The hard drive 52 is coupled to the power supply 50 and the computer 46. The frame grabber 42 is coupled to the power supply 50 and the computer 46.

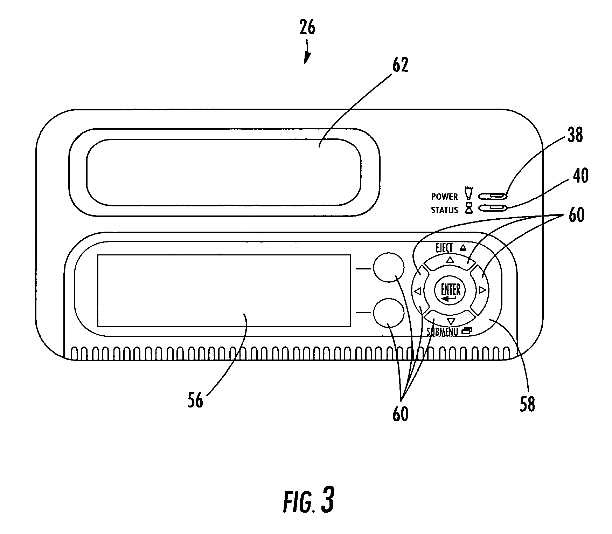

FIG. 3 is a diagram illustrating the front panel user interface 26 that controls operation of the MDR of the present invention. The MDR user interface 26 preferably comprises a LCD display 56 with LED backlighting and a keypad 58 with a plurality of push buttons 60 for controlling operation of the MDR, a power indicator 38, a status indicator 40, and an opening 62 for inserting and removing the removable media from the media writer 44. The LCD display 56 indicates the current status of the MDR After applying power to the MDR, by plugging the AC power connector 66 into an AC power source with a power cord and switching on the power switch 64, the power indicator 38 illuminates and the MDR automatically initializes. Data transfer from a medical image modality to the MDR is initiated by an operator.

FIG. 4 is a diagram illustrating the rear panel 68 of the MDR including the imaging modality interface 28, the network interface 30 and power interface 32. The rear panel 68 also preferably includes a fan 54, an on/off power switch 64, an AC power connector 66, a serial communication connector 68, a mouse connector 70, a keyboard connector 72, a VGA connector 74, a LAN connector 30 for connecting the system to a network, a USB connector 76, and a plurality of video and audio connectors 28. The plurality of video connectors include RGBS connectors 78 for RGBS video in from an imaging modality and RGBS video out to a display, S-Video connectors 80 for S-Video in from an imaging modality and S-Video out to a display, composite video connectors 82 for composite color or monochrome video in from an imaging modality and for composite color or monochrome video out to a display, audio connectors 84 for audio out from an imaging modality and audio in to speakers, and a remote expose connector 86 for connection from an imaging modality or a footswitch of an imaging modality.

The MDR preferably accepts a plurality of analog video inputs and at least two audio channel inputs. Most prior art video recorders are limited to broadcast video formats, such as S-Video of 640 x 480 lines, or color composite sources. The MDR can record the full range of medical video sources up to 1600 x 1200 lines, a 120MHz pixel clock frequency for monochrome video sources and a 50MHz pixel clock frequency for RGB color sources. The MDR can also record interlaced and non-interlaced video sources as well as VGA signals up to a resolution of 1280 x 1024. The MDR is also able to record the broadcast standard formats, such as S-Video and color composite sources. The result of the MDR's broad input range is the playback of images that are identical to the original source and without the drawbacks of scan conversion or reduced image resolution.

The MDR can provide video recording to a DVD without introducing a loss of image quality and output which is equal to that of the original imaging exam data. Each recording removable media (DVD or CD) is preferably recorded in DICOM format from a high resolution video source. The MDR is not limited to recording from lower resolution S-Video or composite video sources. Instead, the imaging data is received and recorded at the highest bandwidth from the imaging modality so that each exam copy is exactly like the original. Images recorded by the MDR are much superior to video tape, S-Video and MPEG images. The superior image quality is particularly beneficial for use in cardiology, angioplasty, vascular and orthopedic applications as well as for any portable imaging modality.

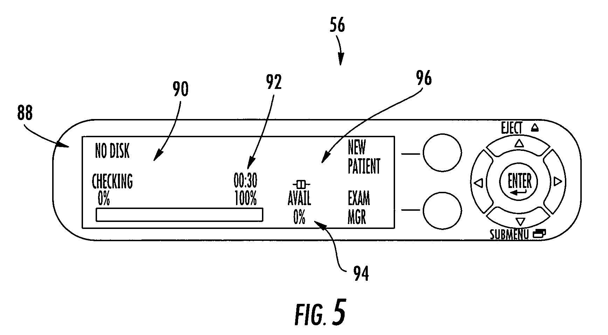

FIG. 5 is a diagram of the display 56 of the medical digital recorder 10 of the present invention. The display 56 indicates the current status of the MDR. For example, the display 56 may include: disk status 88 (no disk, disk inserted and type of disk inserted), MDR activity 90 (no activity, checking media, ejecting disk, cine length, frame rate), estimated time to complete current activity 92, available disk space 94, and whether a video signal is present at the MDR video input 96.

The MDR preferably has at least two modes of operation, an archive mode and a single patient mode. In archive mode, images are captured and automatically written to the removable media. In single patient mode, only images from the selected patient are written to the removable media.

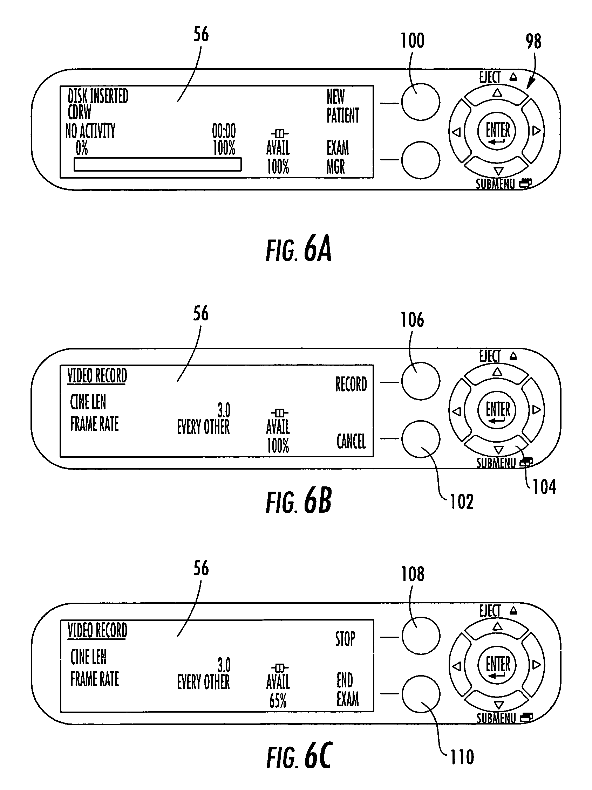

FIGS. 6A-6C are diagrams of the display 56 of the medical digital recorder 10 of the present invention illustrating the recording process during an archive mode. In archive mode, a disk is inserted into the MDR by pressing the eject button on the front panel of the MDR, installing the disk and pressing the disk eject button again. If the disk has not been formatted, the MDR automatically formats the disk. Once the disk has been formatted, the MDR displays the information shown in FIG. 6A. Press the "New Patient" soft key 100 to begin a new exam. Patient data entry is dependent on the MDR's configuration. Once the patient information is confirmed, the MDR is ready to capture images from the connected host. Press the "Cancel" soft key 102 to exit the exam. Press the down arrow button 104 to access the cine setup screen. Press the "Record" soft key 106 to begin capturing images for the new exam. Press the "Stop" soft key 108 to pause recording images. If the "Stop" soft key 108 is not pressed, image capture will stop when the cine length value has been reached. Press the "End Exam" soft key 110 to close the current exam.

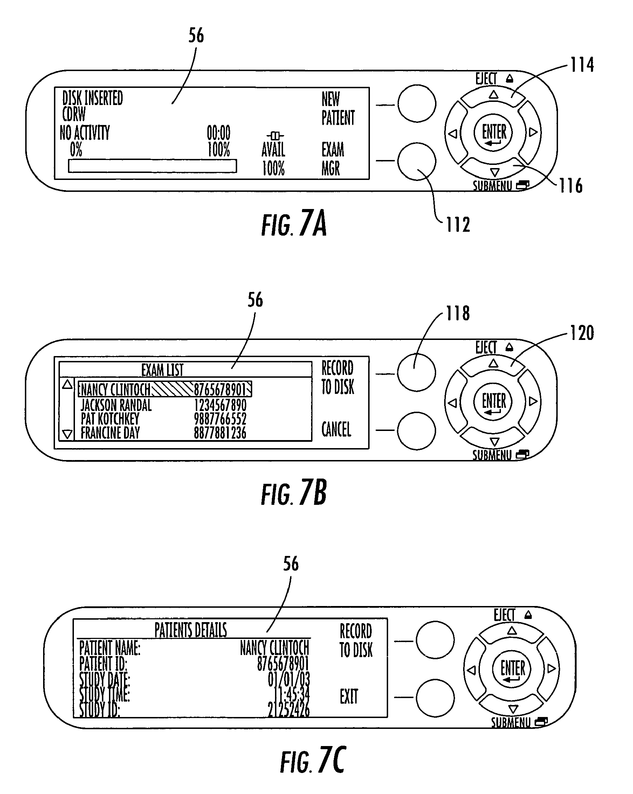

FIGS. 7A-7C are diagrams of the display 56 of the medical digital recorder 10 of the present invention illustrating the recording process during a single patient mode. If the MDR is configured for single patient mode, image files from a single patient are written to the removable media. Press the "Exam Mgr" soft key 112 to view the list of patient exams. Exams are listed in the order that they are received. Use the "Exam Mgr" soft key 112 to view the list of patients on the disk. Use the up 114 or down 116 arrow buttons to search for the patient exam to be written to the removable disk. Once the patient exam is highlighted, press the "Record to Disk" soft key 118 to initialize transfer to the removable disk To view the exam details of a particular patient, highlight the patient name and ID and press the enter button 120.

An MDR utilities menu item may be used to finalize a disk. The utilities menu also contains a plurality f informational screens that can be used to view current MDR setup, serial number, CPU temperature, and software version. To access the utilities menu, press the down arrow key when the MDR display shows the "Disk Writing Process" screen.

To access the MDR cine parameters screen, enter the MDR utilities menu, use the arrow buttons to highlight the "Cine" selection, then press the enter button. Current cine length and frame rate are shown in the cine parameters screen. Use the up and down arrow buttons to select the parameter to be changed. Then press the "Enter" button to edit the parameter. Use the up and down arrow buttons to change the cine length, use the "Enter" button to change the frame rate.

To access the MDR disk utilities function, enter the MDR utilities menu and use the up and down arrow buttons to highlight the "Disk" selection. A "Finalize" screen prompts the operator to "Continue with Finalizing?" Select "OK" to proceed or "Cancel" to exit. The finalizing process may write a viewer application program, such as a DICOM viewer application, to the disk and make the disk compatible with other CD or DVD drives. This viewer application allows viewing DICOM images that have been written to the disk on a personal computer that does not have a DICOM viewer. A log utilities function is also available on the MDR that allows for copying log files to the CD or DVD.

The MDR is configured for operator preferences and video capture via a system setup menu. The system setup menu is accessed by pressing the arrow buttons and the "Enter" button. The MDR must be displaying the "Disk Writing Progress" screen while the buttons are pressed. The left and right arrow buttons are used to navigate through the setup menu. The "Enter" button is used to select the highlighted menu item. The up and down arrow buttons are used to change the selected items value. The "OK" soft key is used to save changes or the "Cancel" soft key is used to exit the setup menu without saving changes.

The MDR of the present invention simplifies the video recording process allowing a user to easily record still images, loops and cine with the touch of a button or with the use of a footswitch. Offering both retrospective and prospective record modes supports the capture of user specified seconds or minutes of image data immediately preceding or following the desired event. The MDR also allows for continuous linear recording of long dynamic runs or one-button capture of single frames directly from streaming video data. The MDR transforms the image recording process as it works efficiently and automatically while a user concentrate on observation and diagnosis.

The MDR recorded disks eliminate the tedious and time consuming review and rewinding process to get to a few seconds of important data. Using the various record modes included with the MDR reduces the amount of non-essential image data captured on a CD or DVD allowing a user to focus on the most crucial clinical data. Also because the MDR records to DVD or CD media, any personal computer may be used as a review station.

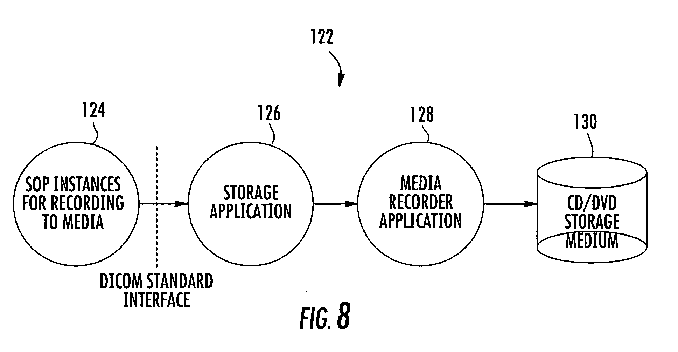

FIG. 8 is an application data flow diagram 122 of the medical digital recorder 10 of the present invention in a DICOM application. The MDR records on CD or DVD media with various DICOM service object pair (SOP) instances 124. The MDR can process various information object definitions (IODs). SOP instances are received via a network as requests from a DICOM application entity (AE), acting as a service class user (SCU), requesting one of various storage service classes. The storage application 126 acts as a service class provider (SCP) for various storage SOP classes. The MDR has local storage utilizing an internal hard drive, which may contain various SOP instances obtained by the storage application 126 via the network. The media recorder application 128 can initialize media by acting as an FSC to create a new DICOM file set on any of the following storage media 130: CD-R, CD-RW, DVD+R and DVD+RW. The MDR is modular in design and may be configured with CD only, DVD only, or a CD/DVD combination drive. The MDR initializes the DICOM file set and writes the specified SOP instances onto the media. The SOP instances written are limited to instances that match the criteria of the application profile that is supported and utilized. The MDR has two application entities: a media recorder application 128 and a storage application 126. The media recorder application 128 initializes a piece of CD/DVD media, and writes a new DICOM file set onto the media. The media recorder application 128 also displays a directory listing of the file set on a piece of CD/DVD media. The storage application 126 acts as a service class provider for various DICOM storage service classes, enabling the MDR to receive SOP instances via the network.

The MDR provides 4.35 GB of storage on a single DVD and up to 40 GB on its internal hard drive. Images can be stored and retrieved without risk of artifacts inherent in tape recording, and may be viewed on any workstation. The MDR also records onto CD as well as DVD media.

The MDR is primarily designed to convert analog video from a source and write it to DVD media in a DICOM format. The MDR recorded media is compatible to DICOM Part 10. allowing medical archives and workstations to directly access the image data. The MDR also installs a DICOM viewer on each disk for review from any personal computer.

The MDR includes various record modes, which are designed to match the workflow of dynamic medical imaging. The MDR can be set by an operator or user to record images in the following modes: retrospective, prospective, sequential or snapshot. All other prior art video recorders are designed to record serially with all images captured in sequence one image after another as they occur in time. The MDR in contrast can be set-up to record at a specific time and frame rate, and when the "Record" button is pressed, write images to a removable recording media on retrospective images (images that have already passed in time) or prospective images (images that will occur in a future time). In addition, dynamic images may be viewed as single frame, high resolution snapshot images, that can be captured as still images without first pausing or freeze framing the source. The MDR can also be used conventionally, by pressing the "Record"," Pause" and "Stop" buttons to capture and record images sequentially.

During the retrospective capture mode, which is the act of recording information after the event has happened. A user can set a capture time of, for example 10 minutes, to creates a 10-minute buffer on the MDR. So, for example, when a user is performing an ultrasound on a patient's liver, the user might be looking for something that takes 25 minutes to find, but once the user has found what they are looking for, the user can stop and record only the most 10 minutes of the exam, instead of the full 25 minutes.

The MDR also includes Automatic Character Recognition (ACR), which is a form of Optical Character Recognition. The ACR allows the patient name, ID number and accession number to be extracted and converted into digital text fields in real time from the video source. The text fields are placed appropriately into the file header data on the removable disk for easy reference, database management and data retrieval during playback. In other words, the ACR identifies the patient name, ID number and accession number from the video information and enters that information into a DICOM file along with the image data. What this means is that the operator does not have to manually enter this information into the system. The data is read off the video signal using a software algorithm built into the MDR system.

The MDR is designed with HIPAA (Health Insurance and Patient Accountability Act) compliance in mind, by providing a number of tools to regulate access to patient images and information. The prior art video recorders have all but ignored HIPAA requirements. The MDR of the present invention incorporates specific HIPAA compliant features requiring operator log-in, password protection, and HIPAA acknowledgement screens on the removable recorded media. The MDR integrates well with any HIPAA implementation, which a healthcare provider may have in place.

While the invention has been described with reference to preferred embodiments, those skilled in the art will appreciate that certain substitutions, alterations and omissions may be made to the embodiments without departing from the spirit of the invention. Accordingly, the foregoing description is meant to be exemplary only, and should not limit the scope of the invention as set forth in the following claims.

## Claims

1. A system for recording medical image data on a digital recording media, the system comprising:
a medical imaging modality interface for receiving a plurality of video signals from a medical imaging modality;
a video capturing device coupled to the medical imaging modality for capturing a plurality of video images from the medical imaging modality and converting the plurality of video images into a plurality of digital images;
a media writer coupled to the video capturing device for recording the plurality of digital images onto a removable media;
a computer having a CPU installed thereon coupled to the medical imaging modality interface, the video capturing device and the media writer for controlling operation of the system; and
a user interface coupled to the computer, the medical imaging modality interface, the video capturing device and the media writer for allowing user input in controlling operation of the system.

2. The system of claim 1, further comprising a network interface for connecting the system to a network.

3. The system of claim 1 or claim 2, further comprising a hard drive for storing a plurality of digital images thereon.

4. The system of any preceding claim further comprising a power interface for connecting the system to a power source.

5. The system of claim 4, further comprising a power supply receiving an input from the power source and providing power to the system.

6. The system of any preceding claim, wherein the medical imaging modality interface comprises one, or more than one, or any number of:-
(i) a plurality of video inputs and video outputs;
(ii) an audio input and audio output;
(iii) a fixed or static medical imaging modality;
(iv) a mobile medical imaging modality.

7. The system of any preceding claim, wherein the plurality of video signals include one, or more than one, or any number of:-
(i) interlaced and non-interlaced video signals;
(ii) RGB video signals;
(iii) S-Video video signals;
(iv) composite color or monochrome video signals;
(v) VGA video signals.

8. The system of any preceding claim, wherein the media writer comprises one of, at least one of, or more than one of:-
(i) a CD writer;
(ii) a DVD writer.

9. The system of any preceding claim, wherein the removable media comprise one of, at least one of, or more than one of:-
(i) a CD;
(ii) a DVD.

10. The system of any preceding claim, wherein the computer includes software installed thereon for controlling operation of the system.

11. The system of claim 10, wherein the software installs a DICOM viewer application onto the removable media for playback.

12. The system of claim 11, wherein the DICOM viewer application includes at least one file export utility, at least one e-mail utility and at least one printing utility.

13. The system of any preceding claim, wherein the user interface includes one of, at least one of, or more than one of:-
(i) a display;
(ii) a keypad with push buttons.

14. A method for recording medical image data on a digital recording media, the method comprising the steps of:
receiving a plurality of video images from a medical imaging modality;
capturing the plurality of video images from the medical imaging modality;
converting the plurality of video images into a plurality of digital images; and recording the plurality of digital images onto a removable media.

15. The method of claim 14, wherein the plurality of video images are digitized and written to a DVD in a medical digital compliant format.

16. The method of claim 14 or claim 15, wherein the medical digital compliant format is a DICOM format.

17. The method of claim 14, wherein the plurality of digital images are recorded in a manner from the group:-
(i) recorded sequentially;
(ii) recorded prospectively;
(iii) recorded retrospectively;
(iv) recorded in a snapshot mode as still images;
(v) recorded at a specific time and a specific frame rate.

18. The method of any of claims 14 to 17, wherein the plurality of video images include one of, at least one of, or more than one of:-
(i) interlaced and non-interlaced video images;
(ii) RGB video images;
(iii) S-Video video images;
(iv) composite color or monochrome video images;
(v) VGA video images.

19. The method of any of claims 14 to 18, further comprising automatically installing a DICOM viewer application onto the removable media.

20. The method of claim 19, further comprising applying automatic character recognition to the plurality of recorded digital images, which allows a patient's name, an ID number and an accession number to be extracted and converted into digital text fields in real time from the plurality of video images.

21. The method of claim 20, wherein the digital text fields are placed into a file header on the removable media for easy reference, database management and data retrieval during playback.

22. The method of any one of claims 14 to 21, further comprising adding HIPAA compliance features to the plurality of recorded digital images including operator log-in, password protection, and HIPAA acknowledgement screens on the recorded removable media.

23. The method of any one of claims 14 to 22, further comprising recording the plurality of digital images onto a removable media via a multiple archive mode that records the plurality of digital images continuously until the removable media is either full or removed by an operator.

24. The method of claim 23, wherein any unrecorded digital images are stored on a hard drive for recording onto a new removable media.

25. The method of any one of claims 14 to 24, further comprising recording a plurality of digital images onto a removable media via a single patient mode that records one patient record per removable media along with a DICOM viewer application,

26. The method of any one of claims 14 to 25, further comprising receiving at least one audio input from the medical imaging modality.

27. The method of any one of claims 14 to 26, further comprising receiving a remote expose from the medical imaging modality or a footswitch.
